# EUROPEAN PATENT SPECIFICATION

(11) **EP 4 287 864 B1**
(45) Date of publication and mention of the grant of the patent: **02.04.2025**
(21) Application number: 22703366.9
(22) Date of filing: 02.02.2022
(51) Int. Cl.: A24F 40/20, A24F 40/46, A61M 11/04, A61M 15/06

(54) **A NON-COMBUSTIBLE AEROSOL PROVISION DEVICE**
VORRICHTUNG ZUR BEREITSTELLUNG EINES AEROSOLS OHNE VERBRENNUNG
DISPOSITIF DE FOURNITURE D'AÉROSOL SANS COMBUSTION

(30) Priority: 03.02.2021 GB 202101469
(43) Date of publication of application: 13.12.2023
(73) Proprietor: Nicoventures Trading Limited, London WC2R 3LA (GB)
(72) Inventor: CAMPBELL, Jeremy, London WC2R 3LA (GB); WRIGHT, Matthew, Cambridge Cambridgeshire CB1 1HZ (GB); WALTON, Daniel Anthony, Cambridge Cambridgeshire CB1 1HZ (GB)
(74) Representative: Dehns
(86) International application number: PCT/EP2022/052467
(87) International publication number: WO 2022/167476

(56) References cited:
- US-A1- 2016 360 794
- US-A1- 2020 054 074

## Description

### Technical Field

The present invention relates to a non-combustible aerosol provision device.

### Background

Smoking articles such as cigarettes, cigars and the like burn tobacco during use to create tobacco smoke. Attempts have been made to provide alternatives to these articles that burn tobacco by creating products that release compounds without burning. Examples of such products are heating devices which release compounds by heating, but not burning, the material. The material may be for example tobacco or other non-tobacco products, which may or may not contain nicotine.

US2020/0054074A1 discloses an adjustable vaporiser that receives atomisers having different dimensions.

### Summary

According to the present invention, as defined in claim 1, there is provided a non-combustible aerosol provision device for generating aerosol from a consumable comprising aerosol-generating material, the non-combustible aerosol provision device comprising: a chamber for receiving a consumable comprising aerosol generating material, the chamber having an inlet and a length, wherein the inlet has a width and wherein the width of the inlet and the length of the chamber are adjustable; and a mechanism for configuring the chamber in at least a first configuration and a second configuration; wherein, when the chamber is in the first configuration the width of the inlet is a first width and the length of the chamber is a first length, and when the chamber is in the second configuration the width of the inlet is a second width that is different from the first width and the length of the chamber is a second length that is different from the first length.

The mechanism comprises a moveable linkage and the mechanism is configured so that a single movement of the moveable linkage causes the chamber to change between the first configuration and the second configuration.

The mechanism may comprise a movable member, the movable member defining a first passage comprising the first width and a second passage comprising the second width and wherein the mechanism is arranged to move the movable member between a first member position corresponding to the first configuration and a second member position corresponding to the second configuration and wherein when the movable member is in the first member position the first passage defines the inlet of the chamber and when the movable member is in the second member position the second passage defines the inlet of the chamber.

The first passage and the second passage may intersect each other in the moveable member.

The movable member may be a rotatable member arranged to rotate relative to the chamber between the first member position and the second member position.

The rotatable member may be rotatable between the first member position and the second member position about a rotation axis that is substantially perpendicular to an axis of the chamber.

The rotation axis may be unaligned with each of the first passage and the second passage when the moveable member is in the first member position and when the moveable member is in the second member position.

The mechanism may comprise a movable base of the chamber for adjusting the length of the chamber.

The mechanism may comprise at least one mechanical linkage connecting the moveable member to the movable base of the chamber.

The at least one mechanical linkage may be arranged so that movement of the movable base from a first base position to a second base position causes the movable member to move from the first member position to the second member position.

The mechanism may convert linear motion of the movable base to rotational motion of the moveable member.

The non-combustible aerosol provision device may further comprise an opening to a recess at a distal end of the non-combustible aerosol provision device, wherein the recess is configured to receive a first removable insert that is insertable by a user into the recess through the opening to push the movable base from the first base position to the second base position.

The non-combustible aerosol provision device may further comprise the first removable insert received within the recess, wherein the first removable insert comprises a material that collects condensate in the recess that forms when the non-combustible aerosol provision device is in use.

The second length of the chamber may be wider than the first length of the chamber.

The second width may be smaller than the first width.

According to the present invention, as defined in claim 11, there is provided a non-combustible aerosol provision device for generating aerosol from a consumable comprising aerosol-generating material, the non-combustible aerosol provision device comprising: a chamber for receiving a consumable comprising aerosol generating material, wherein the inlet has a width that is adjustable; and a mechanism for configuring the chamber in at least a first configuration and a second configuration; wherein, when the chamber is in the first configuration the width of the inlet is a first width and when the chamber is in the second configuration the width of the inlet is a second width that is different from the first width, wherein the mechanism comprises a movable member, the movable member defining a first passage comprising the first width and a second passage comprising the second width and wherein the mechanism is arranged to move the movable member between a first member position corresponding to the first configuration and a second member position corresponding to the second configuration and wherein when the movable member is in the first member position the first passage defines the inlet of the chamber and when the movable member is in the second member position the second passage defines the inlet of the chamber.

The first passage and the second passage may intersect each other in the moveable member.

The movable member may be a rotatable member arranged to rotate relative to the chamber between the first member position and the second member position.

The rotatable member may be rotatable between the first member position and the second member position about a rotation axis that is substantially perpendicular to an axis of the chamber.

The rotation axis may be unaligned with each of the first passage and the second passage when the moveable member is in the first member position and when the moveable member is in the second member position.

The chamber may further comprise an adjustable length and the mechanism may comprise a movable base of the chamber for adjusting the length of the chamber.

The mechanism may comprise at least one mechanical linkage connecting the moveable member to the movable base of the chamber.

The at least one mechanical linkage may be arranged so that movement of the movable base from a first base position to a second base position causes the movable member to move from the first member position to the second member position.

In accordance with a third aspect of some embodiments described herein, there is provided a non-combustible aerosol provision system comprising: a non-combustible aerosol provision device according to the first aspect or to the second aspect; and at least one consumable comprising aerosol-generating material, the consumable for being received in the chamber.

Further features and advantages of the invention will become apparent from the following description of preferred embodiments of the invention, given by way of example only, which is made with reference to the accompanying drawings.

### Brief Description of the Drawings

Figure 1 shows a schematic diagram of a system comprising a non-combustible aerosol provision device and a consumable comprising an aerosol generating material;
Figure 2 shows a schematic perspective view of the non-combustible aerosol provision device when a chamber mechanism is configured in a first configuration;
Figure 3 shows a perspective view of the non-combustible aerosol provision device when the chamber mechanism is in a second configuration;
Figure 4a shows a schematic close-up perspective view of part of the non-combustible aerosol provision device when the chamber mechanism is configured in the first configuration;
Figure 4b shows a schematic close-up perspective view of part of the non-combustible aerosol provision device when the chamber mechanism is configured in a second configuration;
Figure 5a shows a schematic perspective view of the non-combustible aerosol provision device when the chamber mechanism is transitioning from the first configuration to the second configuration;
Figure 5b shows a schematic perspective view of the non-combustible aerosol provision device when the chamber mechanism is transitioning from the first configuration to the second configuration;
Figure 6a shows a schematic perspective view of the non-combustible aerosol provision device when the chamber mechanism is transitioning back from the second configuration to the first configuration;
Figure 6b shows a schematic perspective view of the non-combustible aerosol provision device when the chamber mechanism is transitioning back from the second configuration to the first configuration;
Figure 7 shows a schematic perspective view of a lower portion of the non-combustible aerosol provision device.

### Detailed Description

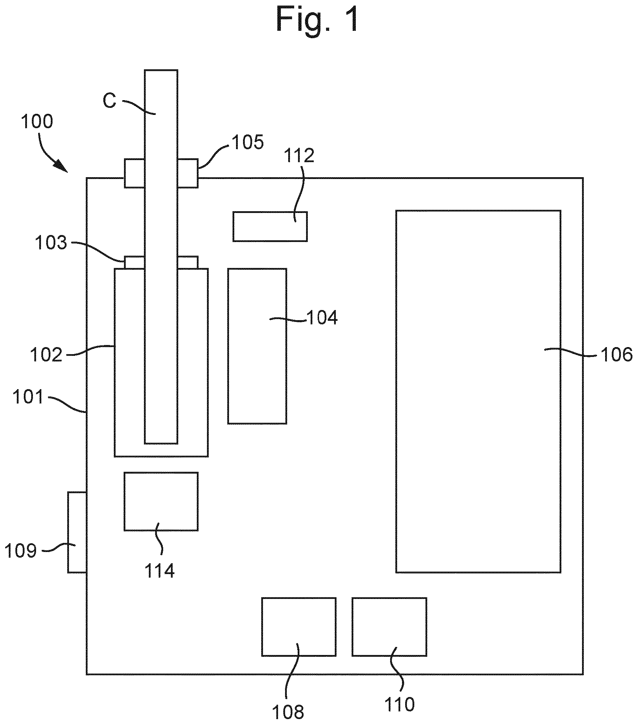

Figure 1 is a simplified schematic view of a non-combustible aerosol provision device 100. The non-combustible aerosol provision device 100 comprises a housing 101 that houses the various components of the non-combustible aerosol provision device 100. The non-combustible aerosol provision device 100 comprises a chamber 102 configured to receive a consumable (C) which comprise aerosol generating material.

Aerosol-generating material is a material that is capable of generating aerosol, for example when heated, irradiated or energized in any other way. Aerosol-generating material may, for example, be in the form of a solid, liquid or gel which may or may not contain an active substance and/or flavourants. In some embodiments, the aerosol-generating material may comprise an "amorphous solid", which may alternatively be referred to as a "monolithic solid" (i.e. non-fibrous). In some embodiments, the amorphous solid may be a dried gel. The amorphous solid is a solid material that may retain some fluid, such as liquid, within it. In some embodiments, the aerosol-generating material may for example comprise from about 50wt%, 60wt% or 70wt% of amorphous solid, to about 90wt%, 95wt% or 100wt% of amorphous solid.

The aerosol-generating material may comprise one or more active substances and/or flavours, one or more aerosol-former materials, and optionally one or more other functional material.

An active substance may be a physiologically active material, which is a material intended to achieve or enhance a physiological response. The active substance may for example be selected from nutraceuticals, nootropics, psychoactives. The active substance may be naturally occurring or synthetically obtained. The active substance may comprise for example nicotine, caffeine, taurine, theine, vitamins such as B6 or B12 or C, melatonin, cannabinoids, or constituents, derivatives, or combinations thereof. The active substance may comprise one or more constituents, derivatives or extracts of tobacco, cannabis or another botanical.

In some examples, the active substance comprises nicotine. In some embodiments, the active substance comprises caffeine, melatonin or vitamin B 12.

The aerosol-former material may comprise one or more constituents capable of forming an aerosol. In some embodiments, the aerosol-former material may comprise one or more of glycerol, propylene glycol, diethylene glycol, triethylene glycol, tetraethylene glycol, 1,3-butylene glycol, erythritol, meso-Erythritol, ethyl vanillate, ethyl laurate, a diethyl suberate, triethyl citrate, triacetin, a diacetin mixture, benzyl benzoate, benzyl phenyl acetate, tributyrin, lauryl acetate, lauric acid, myristic acid, and propylene carbonate
The one or more other functional materials may comprise one or more of pH regulators, colouring agents, preservatives, binders, fillers, stabilizers, and/or antioxidants.

The non-combustible aerosol provision device 100 is herein after referred to as the device 100. The device 100 comprises an aerosol generator 104 for causing aerosol to be generated from the aerosol generating material in a consumable received in the chamber 102.

An aerosol generator is an apparatus configured to cause aerosol to be generated from aerosol-generating material. In some embodiments, the or each aerosol generator is a heater configured to subject aerosol-generating material to heat energy, so as to release one or more volatiles from the aerosol-generating material to form an aerosol. In some embodiments, the or each aerosol generator is configured to cause an aerosol to be generated from the aerosol-generating material without heating. For example, the aerosol generator may be configured to subject the aerosol-generating material to one or more of vibration, increased pressure, or electrostatic energy.

Accordingly, in some examples the aerosol generator 104 comprises a heating arrangement configured to provide energy for heating the aerosol generating material in the consumable. In some examples, the heating arrangement comprises one or more resistive heating elements arranged in thermal contact with the chamber 102. The flow of current against the electrical resistance of the one or more resistive heating elements generates heat. This process is called Joule, ohmic, or resistive heating.

In some examples, the aerosol generator 104 is an induction heating arrangement and is configured to generate a varying magnetic field in order to inductively heat a susceptor. An induction heating arrangement may comprise one or more inductors through which an alternating current is passed to generate the varying magnetic field. In some example, the aerosol generator 104 comprises one or more susceptors. In other examples, the aerosol generator 104 may not comprise a susceptor and one or more susceptors may instead be provided as part of/with consumable articles intended for use with the device 100.

A susceptor is a material that is heatable by penetration with a varying magnetic field, such as an alternating magnetic field. The susceptor may be an electrically-conductive material, so that penetration thereof with a varying magnetic field causes induction heating of the heating material. The heating material may be magnetic material, so that penetration thereof with a varying magnetic field causes magnetic hysteresis heating of the heating material. The susceptor may be both electrically-conductive and magnetic, so that the susceptor is heatable by both heating mechanisms. A device that is configured to generate the varying magnetic field is referred to as a magnetic field generator, herein.

The device 100 comprises a power source 106. The power source 106 supplies electrical power to the various components of the device 100. In some examples, the power source 106 is a battery. In some examples, the power source 106 comprises a battery and a DC-DC converter, and power is supplied from the battery through the DC-DC converter. The DC-DC converter may allow the power supply 106 to supply power at a different voltage to the voltage of the battery. In some examples, the device 100 may comprise a DC to AC converter for converting a DC current from e.g. a battery to AC current, for example, to supply power to one or more inductors of the heating arrangement 104 where the heating arrangement 104 is an induction heating arrangement. In the following examples, the power source 106 is referred to simply as the battery 106.

In the example of Figure 1, the device 100 comprises control circuitry 108 configured to control various aspects of the operation of the device 100 including the aerosol generator 104. In this example, the control circuitry 108 is a control processor 108 in data communication with a computer readable memory 110. The control circuitry 108 controls the various operations of the device, for example, by executing instructions stored on the computer readable memory 110. For example, the control circuitry 108 may control the delivery of electrical power from the battery 106 to the aerosol generator 104 by controlling various electrical component such as switches and the like (not shown in Figure 1).

The device 100 further comprises a user input device 112 which is electrically connected to the control circuitry 108 and by means of which a user can operate the device 100. Examples of a suitable user input device 109 include one or more buttons, a track pad and a touch screen although any suitable device may be used.

In this example, the device 100 also comprises an opening 105 at one end of the housing 101 which opens into an inlet 103 of the chamber 102. A user can insert a consumable into the chamber 102 through the opening 105 such that a distal portion of the consumable is received within the chamber 102 whilst a proximal portion of the consumable protrudes from the device 100.

The distal portion of the consumable that is received in the chamber 102 comprises the aerosol generating material from which the device 100 is configured to generate aerosol. The proximal portion of the consumable which protrudes from the opening 105 may, for example, comprise a filter or the like, and the user may insert the proximal portion into their mouth in order to inhale a flow of aerosol when the device 100 is being used.

In this example, the inlet 103 of the chamber 102 comprises an adjustable width and the chamber 102 comprises an adjustable axial length. To that end, the device 100 comprises a mechanism 114 for configuring the chamber 102 in at least a first configuration and a second configuration. When the chamber 102 is in the first configuration the inlet 103 has a first width and the chamber 102 has a first axial length whereas when the chamber 102 is in the second configuration the inlet 103 has a second width and the chamber 102 has a second axial length.

Advantageously therefore, the device 100 is easily configurable to be used with at least two different types of consumables that provide a different user experience to users and are of different sizes.

It should be noted that, herein, reference to consumables of different sizes refers to consumables which are intended to be a different size to another, and does not, for example, refer to consumables which are intended to be the same size but differ in size by some small amount due to, e.g., manufacturing tolerances.

In one example, the first axial length is longer than the second axial length and the first width is smaller than the second width. This means than when the chamber 102 is in the first configuration the device 100 is for use with a first type of consumable that is longer and narrower than a second type of consumable that the device 100 is for use when the chamber 102 is in the second configuration.

It will be appreciated that the device 100 may comprise other components not shown in Figure 1, such as ventilation inlets/outlet, a control interface, a charging port, etc. It should be noted that Figure 1 is merely a schematic sketch showing a number of components that may be included in the device 100. Figure 1 is not intended to communicate particular positions of various components.

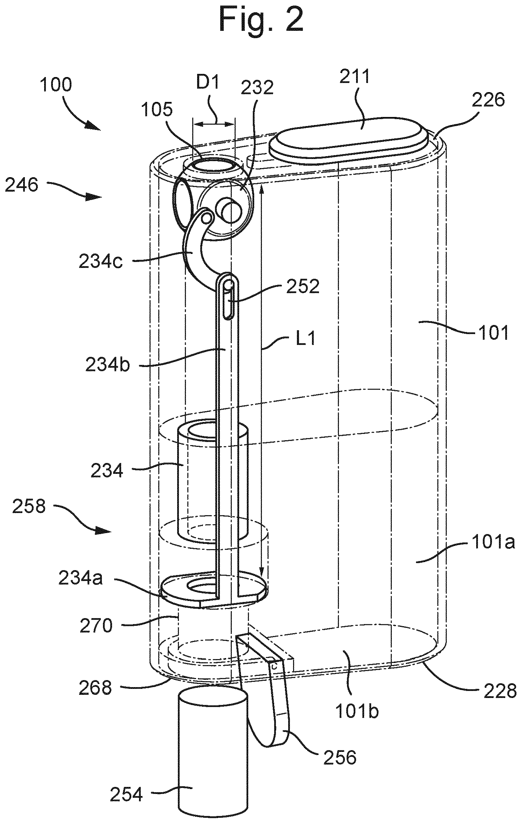

Figures 2 to 7 illustrate an example of the device 100 with the housing 101 in ghosted form (i.e. the housing 101 is falsely illustrated as see through) so as to reveal some of the internal components of the device 100. For reasons of clarity, none of the power supply 106, processor 108 or memory 110 are shown in these Figures.

The housing 101 comprises a plurality of panels including a front panel 226 at the proximal end of the device 100, a rear panel 228 at the distal end of the device, a first side panel 101a and a second side panel 101b. The first side panel and the second side are 'U' shaped in cross section and connect together, for example as a 'snap fit', to define the sides of the housing 101. The front panel 226 and the bottom panel 228 are connected to the first side panel and the second side at opposite ends of the housing 101 to define the front and rear of the housing 101 respectively.

Typically, each of the plurality panels comprises a plastics material, or any other type of suitable material. Typically, each panel may be formed by injection moulding.

The front panel 226 defines the opening 105 which communicates with the inlet 103 of the chamber 102. The front panel 226 has a slidable cover 211 that slides over the opening 105 so that the chamber 102 can be closed when the device 100 is not in use.

The chamber 102 is generally cylindrical in form and extends parallel to the axial axis of the device 100.

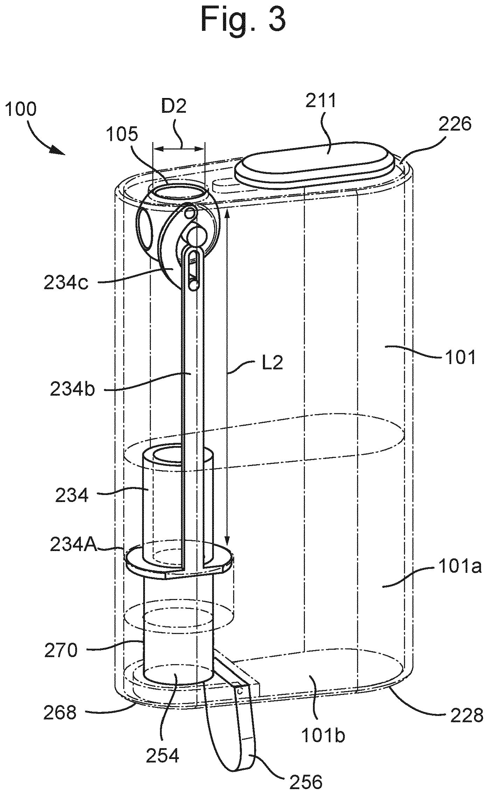

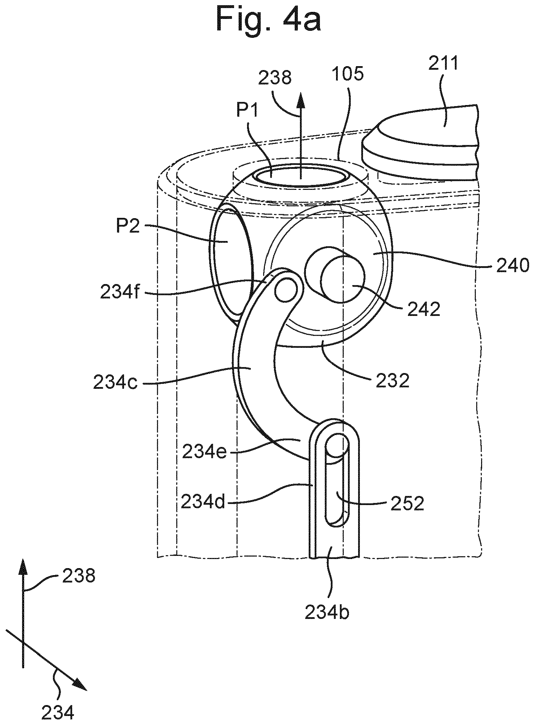

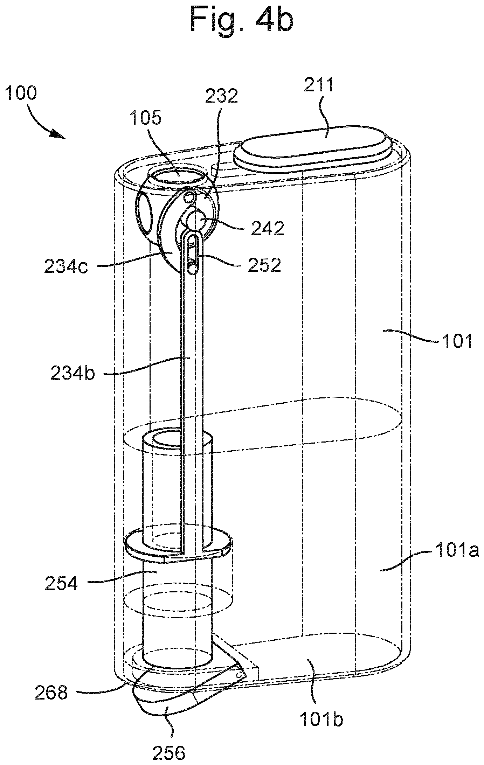

Figures 2 and 4a illustrate the device 100 when the chamber 102 is configured in the first configuration and Figures 3 and 4b illustrate the device 100 when the chamber 102 is configured in the second configuration.

In the first configuration, the chamber 101 has a first axial length L1 and the inlet 103 to the chamber has a first width D1. In the second configuration, the chamber 101 has a second axial length L2 (which is shorter than L1) and the inlet 103 to the chamber has a second width D2 (which is wider than D1).

The mechanism 114 for configuring the chamber 101 from the first configuration to the second configuration (and vice versa) comprises a moveable, in this example rotatable, member 232 and a frame 234 that comprises a base portion 234a, an elongate link arm 234b and a curved link arm 234c. In this example, the base portion 234a is annular.

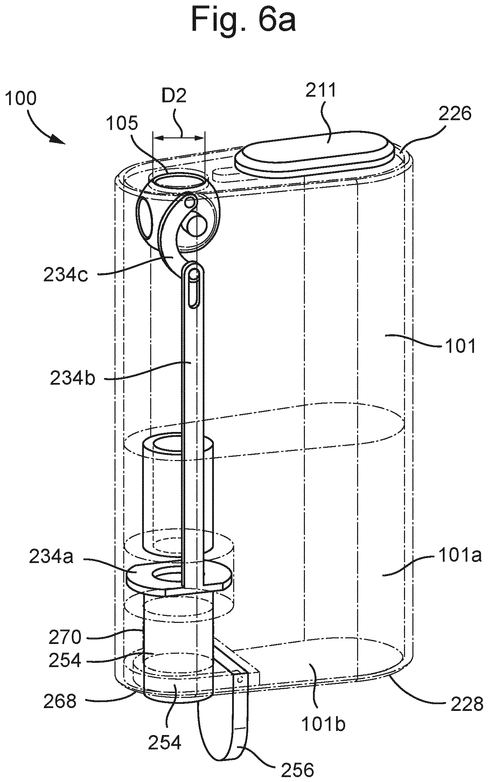

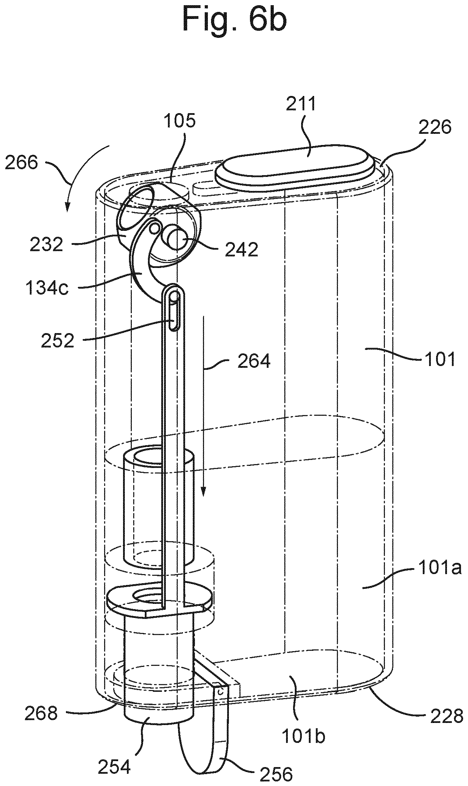

The rotatable member 232 is rotatably mounted about axes of a pair of collinear shafts 242 (of which only one is visible in the Figures). In some examples, the shafts 242 are discrete from the rotatable member 232 and are fixed, one either side of the rotatable member 232, to the interior of the housing 101 below the opening 105. Alternatively, in some examples, the shafts 242, again one either side of the rotatable member 232, are integrally formed with the rotatable member 232 and are rotatably fixed, for example, to respective sockets (not shown) in the interior of the housing 101 below the opening 105. The collinear axes of the shafts 242 are substantially perpendicular to the axial axis of the chamber 102 as indicated by arrows 234 and 238 in Figure 6. In this example, the rotatable member 232 is roughly cuboidal in shape but has rounded edges. The rotatable member 232 defines a first open ended passageway P1 that extends through the rotatable member 232 between a first pair of opposite faces of the rotatable member 232 and also defines a second open ended passageway P2 that extends through the rotatable member 232 between a second pair of opposite faces of the rotatable member 232. The rotatable member 232 is rotatably mounted on one of the shafts 242 at one of 240 a third pair of opposite faces of the rotatable member 232 and is rotatably mounted on the other of the shafts 242 (not visible in the figures) at the other (not visible in the Figures) of the third pair of opposite faces of the rotatable member 232.

The first P1 and second P2 open ended passageways are perpendicular to each other and also to the axes of the shafts 242 and intersect each other in the rotatable member 232. The first open ended passageway P1 defines the first width D1 and the second passageway P2 defines the second width D2.

When the chamber 101 is in the first configuration, the rotatable member 232 is rotated so that the first open ended passageway P1 is co-axial with the axial axis of the chamber 102 and with the opening 105. Hence when the chamber 101 is in this configuration, the first width D1 of the first open ended passageway P1 defines the first width of the inlet 103 of the chamber 102. When the chamber is in the second configuration, the rotatable member 232 is rotated so that the second open ended passageway P2 is co-axial with the axial axis of the chamber 102 and with the opening 105. Hence when the chamber 102 is in this configuration, the second width D2 of the second open ended passageway P2 defines the second width of the inlet 103 of the chamber 102.

The base portion 234a is in the form of an annular support ring arranged coaxially with the axial axis of the chamber 102. The base portion 234a is moveable from a first base portion position shown in Figure 2 when the chamber 102 is configured in the first configuration to a second base portion position shown in Figure 3 when the chamber 102 is configured in the second configuration.

The elongate link arm 234b is connected at a first end to the base portion 234a and extends from the base portion 234a parallel to the axial axis of the chamber 102. As best shown in Figures 4a and 4b, a second end 234d of the elongate link arm 234b defines an elongate slot 252 within which a first end 234e of the curved link arm 234c is pivotally coupled. This coupling allows the elongate link arm 234b to slide across the first end of the curved link arm 234c by a distance defined by the upper and lower ends of the slot 252. A second end 234f of the curved link arm 234c is pivotably mounted to the face of the rotatable member 232 by a pivot 248.

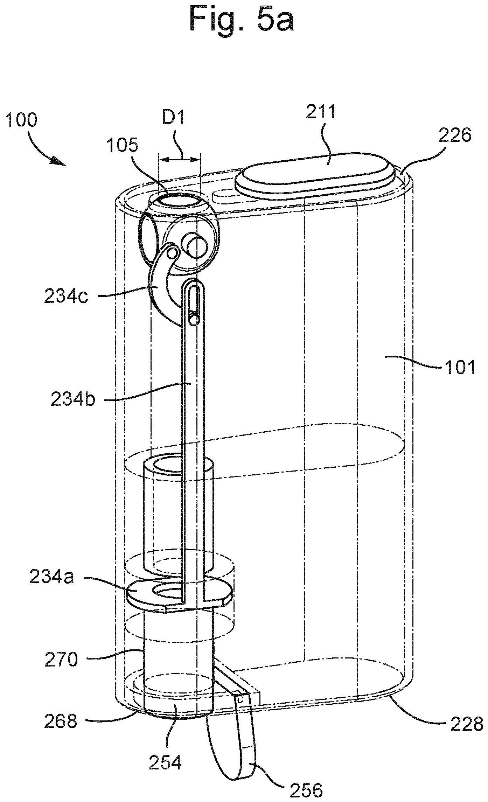

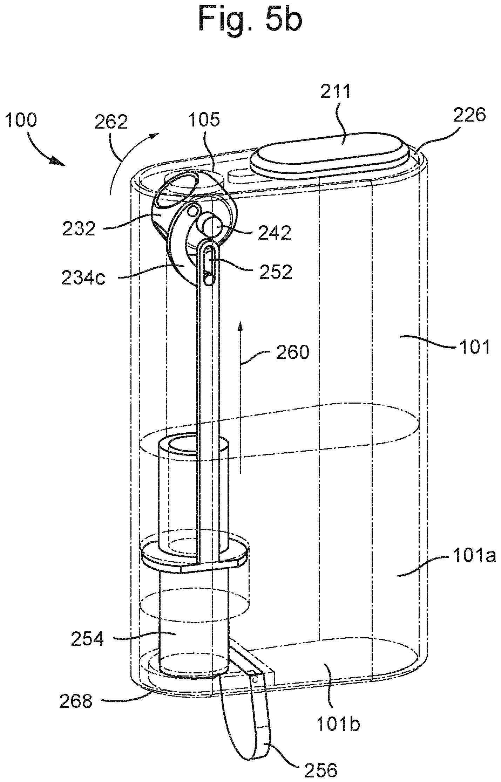

When the base portion 234a is moved from the first base portion position shown in Figure 2 to the second base portion position shown in Figure 3, there is an initial period during which the elongate link arm 234b slides over the first end of the curved link arm 234c which is within the slot 252 and the rotatable member 232 remains stationary because no force is transmitted to the rotatable member 232. This is shown best in Figure 5a where the base portion 234a is between the first base portion position and second base portion position. When the movement of the base portion 234a and the elongate link arm 234b brings the lower end of the slot 252 into contact with the curved link arm 234c the continued movement of the base portion 234a and the elongate link arm 234b (indicated by arrow 260 on Figure 5b) then causes the curved link arm 234c to exert a turning force (indicated by curved arrow 262 on Figure 5b) on the rotatable member 232 which rotates from the first rotatable member position to the second rotatable member position as the base portion 234a completes its movement to the second base portion position.

Similarly, when the base portion 234a is moved back from the second base portion position shown in Figure 3 to the first base portion position shown in Figure 2, there is an initial period when the rotatable member 232 remains stationary as the elongate link arm 234b slides over the first end of the curved link arm 234c until the upper end of the slot 252 contacts the curved link arm 234c. This is shown best in Figure 6a. The continued movement of the base portion 234a and the elongate link arm 234b (shown by arrow 264 on Figure 6b) then causes the curved link arm 234c to exert a turning force (shown by curved arrow 266 on Figure 6b) on the rotatable member 232 which rotates from the second rotatable member position to the first rotatable member position as the base portion 234a completes its movement to the first base portion position.

It will be appreciated that in this example, the rotatable member 232 rotates about a rotation axis that is substantially perpendicular to the axial axis of the chamber 102. Furthermore, the rotation axis is substantially perpendicular to (and hence unaligned with) each of the first open ended passageway P1 and the second open ended passageway P2 whether the rotatable member 232 is in the first member position or in the second member position.

The device 100 further comprises a second opening 268 formed through the rear panel 228 to provide access to a lower internal chamber 270 that is to the rear of and coaxial with the chamber 102. The device 100 comprises a door 256 hinged on the rear panel 228 for opening and closing the opening. The lower internal chamber 270 has a variable internal size.

In this example, in order to configure the chamber 102 from the first configuration to the second configuration, a user may insert a first member 254 into the lower internal chamber 270 to engage the base portion 234a and then push the base portion 234a from the first base portion position to the second base portion position. The first member 254 is shaped so that when the chamber 102 is in the second configuration and the door 256 is closed, the first member 254 sits snuggly in the lower internal chamber 270 between the base portion 234a and the door 256.

In some examples, the base portion 234a is biased towards the first base portion 234a position. In order to configure the chamber 102 from the second configuration to the first configuration, a user removes the first member 254 from the internal chamber 270 and the biased base portion 234a returns to the first base portion 234a position.

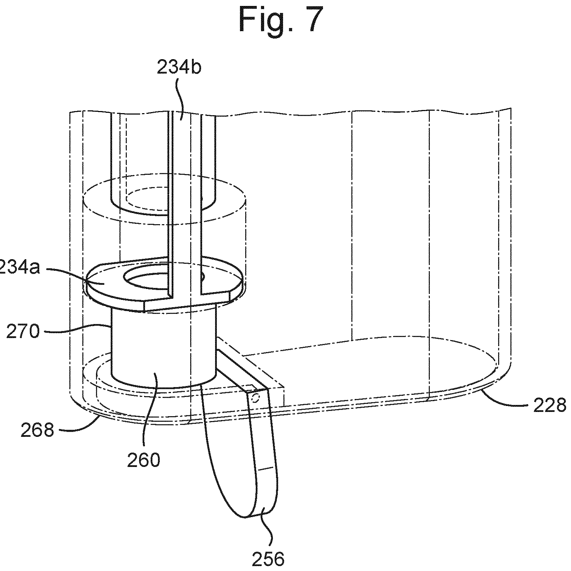

When the chamber 102 is to be used in the first configuration, a user may insert a second member 260 into the lower internal chamber 270 to rest against the first base portion 234a. The second elongate member 260 is shaped so that when the heating chamber 102 is in the first configuration and the door 256 is closed, the second elongate member 260 sits snuggly in the lower internal chamber 270 between the base portion 234a and the door 256. This is shown best in Figure 7.

The first member 254 may comprise material of the type typically used in filters for consumables, e.g. fibrous material such as cellulose acetate. When the first member 254 is in the lower internal chamber 270 and the device 100 is use the first member 254 may adsorb or otherwise collect condensate in the lower internal chamber 270. Similarly, the second member 260 may also comprise material of the type typically used in filters and may perform a condensate collection role when located in the lower internal chamber 270.

In some examples the chamber 102 is accessible through the second opening 268 formed through the rear panel 228, the lower internal chamber 270 and the annular base in part 234a, for cleaning. Furthermore, in some examples, there are one or more holes (not shown) in or around the door 256 for the inlet of air in use when a user draws on a consumable received in the chamber 102. This air flows through the annular base part 234a and into an axially-central part of the consumable.

In the example described above, the larger, second passageway is configured to be used when the chamber 102 is shortest. However, it will be appreciated that this arrangement could be different in an alternative embodiment i.e. the second passageway is used when the chamber 102 is longest. Furthermore, other methods of moving elongate link arm 234b and rotating the rotatable member 232, could be envisaged. For example, the base portion 234a may be moved by a screw thread or with a lever. The rotatable member 232 could also be rotated without the mechanism shortening the length of the heating chamber 102, thus achieving a device that can accommodate two different diameter consumable articles having the same length. This could be achieved in a number of ways, for example, by way of rotating a twist knob on the side of the device.

The above embodiments are to be understood as illustrative examples of the invention. It is to be understood that any feature described in relation to any one embodiment may be used alone, or in combination with other features described, and may also be used in combination with one or more features of any other of the embodiments, or any combination of any other of the embodiments.

## Claims

1. A non-combustible aerosol provision device (100) for generating aerosol from a consumable (C) comprising aerosol-generating material, the non-combustible aerosol provision device (100) comprising:
a chamber (102) for receiving a consumable (C) comprising aerosol generating material, the chamber (102) having an inlet (103) and a length, wherein the inlet (103) has a width and wherein the width of the inlet (103) and the length of the chamber (102) are adjustable; and
a mechanism (114) for configuring the chamber (102) in at least a first configuration and a second configuration;
wherein, when the chamber (102) is in the first configuration the width of the inlet (103) is a first width (D1) and the length of the chamber (102) is a first length (L1), and when the chamber (102) is in the second configuration the width of the inlet (103) is a second width (D2) that is different from the first width (D1) and the length of the chamber (102) is a second length (L2) that is different from the first length (L1);
**characterised in that** the mechanism (114) comprises a moveable linkage (234b, 234c) and wherein the mechanism (114) is configured so that a single movement of the moveable linkage (234b, 234c) causes the chamber (102) to change between the first configuration and the second configuration.

2. The non-combustible aerosol provision device (100) according to claim 1, wherein the mechanism (114) comprises a movable member (232), the movable member (232) defining a first passage (P1) comprising the first width (D1) and a second passage (P2) comprising the second width (D2) and wherein the mechanism (114) is arranged to move the movable member (232) between a first member position corresponding to the first configuration and a second member position corresponding to the second configuration and wherein when the movable member (232) is in the first member position the first passage (P1) defines the inlet (103) of the chamber (102) and when the movable member (232) is in the second member position the second passage (P2) defines the inlet (103) of the chamber (102);
optionally wherein the first passage (P1) and the second passage (P2) intersect each other in the moveable member (232).

3. The non-combustible aerosol provision device (100) according to claim 2, wherein the movable member (232) is a rotatable member arranged to rotate relative to the chamber (102) between the first member position and the second member position;
optionally wherein the rotatable member is rotatable between the first member position and the second member position about a rotation axis that is substantially perpendicular to an axis of the chamber (102);
optionally wherein the rotation axis is unaligned with each of the first passage (P1) and the second passage (P2) when the moveable member (232) is in the first member position and when the moveable member (232) is in the second member position.

4. The non-combustible aerosol provision device (100) according to any one of claims 2 or 3, wherein the mechanism (114) comprises a movable base (234a) of the chamber (102) for adjusting the length of the chamber (102).

5. The non-combustible aerosol provision device (100) according to claim 4, wherein the mechanism (114) comprises at least one mechanical linkage (234b, 234c) connecting the moveable member (232) to the movable base (234a) of the chamber (102).

6. The non-combustible aerosol provision device (100) according to claim 5, wherein the at least one mechanical linkage (234b, 234c) is arranged so that movement of the movable base (234a) from a first base position to a second base position causes the movable member (232) to move from the first member position to the second member position.

7. The non-combustible aerosol provision device (100) according to any one of claims 4 to 6, wherein the mechanism (114) converts linear motion of the movable base (234a) to rotational motion of the moveable member (232).

8. The non-combustible aerosol provision device (100) according to any one of claims 4 to 7, further comprising an opening (268) to a recess (270) at a distal end of the non-combustible aerosol provision device (100), wherein the recess (270) is, configured to receive a first removable insert (254) that is insertable by a user into the recess (270) through the opening (268) to push the movable base (234a) from the first base position to the second base position;
optionally further comprising the first removable insert (254) received within the recess (270), wherein the first removable insert (254) comprises a material that collects condensate in the recess (270) that forms when the non-combustible aerosol provision device (100) is in use.

9. The non-combustible aerosol provision device (100) according to any one of claims 1 to 8, wherein the second length (L2) of the chamber (102) is greater than the first length (L1) of the chamber (102).

10. The non-combustible aerosol provision device (100) according to any one of claims 1 to 9, wherein the second width (D2) is smaller than the first width (D1).

11. A non-combustible aerosol provision device (100) for generating aerosol from a consumable (C) comprising aerosol-generating material, the non-combustible aerosol provision device (100) comprising:
a chamber (102) for receiving a consumable (C) comprising aerosol generating material, wherein the chamber (102) has an inlet (103) which has a width that is adjustable; and
a mechanism (114) for configuring the chamber (102) in at least a first configuration and a second configuration;
wherein, when the chamber (102) is in the first configuration the width of the inlet (103) is a first width (D1) and when the chamber (102) is in the second configuration the width of the inlet (103) is a second width (D2) that is different from the first width (D1), wherein the mechanism (114) comprises a movable member (232),
**characterised in that**
the movable member (232) defines a first passage (P1) comprising the first width (D1) and a second passage (P2) comprising the second width (D2) and wherein the mechanism (114) is arranged to move the movable member (232) between a first member position corresponding to the first configuration and a second member position corresponding to the second configuration and wherein when the movable member (232) is in the first member position the first passage (P1) defines the inlet (103) of the chamber (102) and when the movable member (232) is in the second member position the second passage (P2) defines the inlet (103) of the chamber (102).

12. The non-combustible aerosol provision device (100) according to claim 11, wherein the first passage (P1) and the second passage (P2) intersect each other in the moveable member (232).

13. The non-combustible aerosol provision device (100) according to claim 11 or claim 12, wherein the movable member (232) is a rotatable member arranged to rotate relative to the chamber (102) between the first member position and the second member position;
optionally wherein the rotatable member (232) is rotatable between the first member position and the second member position about a rotation axis that is substantially perpendicular to an axis of the chamber (102);
optionally wherein the rotation axis is unaligned with each of the first passage (P1) and the second passage (P2) when the moveable member (232) is in the first member position and when the moveable member (232) is in the second member position.

14. The non-combustible aerosol provision device (100) according to any one of claims 11 to 13, wherein the chamber (102) further comprises an adjustable length and the mechanism (114) comprises a movable base (234a) of the chamber (102) for adjusting the length of the chamber (102);
optionally wherein the mechanism (114) comprises at least one mechanical linkage (234b, 234c) connecting the moveable member (232) to the movable base (234a) of the chamber;
optionally wherein the at least one mechanical linkage (234b, 234c) is arranged so that movement of the movable base (234a) from a first base position to a second base position causes the movable member (232) to move from the first member position to the second member position.

15. A non-combustible aerosol provision system comprising:
a non-combustible aerosol provision device (100) according to any one of claims 1 to 14; and
at least one consumable (C) comprising aerosol-generating material, the consumable (C) for being received in the chamber (102).

## Patentansprüche

1. Vorrichtung zur Bereitstellung eines nicht brennbaren Aerosols (100) zum Erzeugen eines Aerosols aus einem Verbrauchsmaterial (C), das Aerosol erzeugendes Material umfasst, wobei die Vorrichtung zur Bereitstellung eines nicht brennbaren Aerosols (100) umfasst:
eine Kammer (102) zum Aufnehmen eines Verbrauchsmaterials (C), das Aerosol erzeugendes Material umfasst, wobei die Kammer (102) einen Einlass (103) und eine Länge aufweist, wobei der Einlass (103) eine Breite aufweist und wobei die Breite des Einlasses (103) und die Länge der Kammer (102) angepasst werden können; und
einen Mechanismus (114) zum Konfigurieren der Kammer (102) in mindestens einer ersten Konfiguration und einer zweiten Konfiguration;
wobei, wenn sich die Kammer (102) in der ersten Konfiguration befindet, die Breite des Einlasses (103) eine erste Breite (D1) ist und die Länge der Kammer (102) eine erste Länge (L1) ist, und wenn sich die Kammer (102) in der zweiten Konfiguration befindet, die Breite des Einlasses (103) eine zweite Breite (D2) ist, die sich von der ersten Breite (D1) unterscheidet, und die Länge der Kammer (102) eine zweite Länge (L2) ist, die sich von der ersten Länge (L1) unterscheidet;
**dadurch gekennzeichnet, dass** der Mechanismus (114) eine bewegliche Verbindung (234b, 234c) umfasst, und wobei der Mechanismus (114) so konfiguriert ist, dass eine einzige Bewegung der beweglichen Verbindung (234b, 234c) bewirkt, dass die Kammer (102) zwischen der ersten Konfiguration und der zweiten Konfiguration wechselt.

2. Vorrichtung zur Bereitstellung eines nicht brennbaren Aerosols (100) nach Anspruch 1, wobei der Mechanismus (114) ein bewegliches Element (232) umfasst, wobei das bewegliche Element (232) einen ersten Durchgang (P1), der die erste Breite (D1) umfasst, und einen zweiten Durchgang (P2) definiert, der die zweite Breite (D2) umfasst, und wobei der Mechanismus (114) so angeordnet ist, dass er das bewegliche Element (232) zwischen einer ersten Elementposition, die der ersten Konfiguration entspricht, und einer zweiten Elementposition, die der zweiten Konfiguration entspricht, bewegt, und wobei, wenn sich das bewegliche Element (232) in der ersten Elementposition befindet, der erste Durchgang (P1) den Einlass (103) der Kammer (102) definiert, und wenn sich das bewegliche Element (232) in der zweiten Elementposition befindet, der zweite Durchgang (P2) den Einlass (103) der Kammer (102) definiert;
gegebenenfalls wobei sich der erste Durchgang (P1) und der zweite Durchgang (P2) in dem beweglichen Element (232) kreuzen.

3. Vorrichtung zur Bereitstellung eines nicht brennbaren Aerosols (100) nach Anspruch 2, wobei das bewegliche Element (232) ein drehbares Element ist, das so angeordnet ist, dass es sich relativ zu der Kammer (102) zwischen der ersten Elementposition und der zweiten Elementposition dreht;
gegebenenfalls wobei das drehbare Element zwischen der ersten Elementposition und der zweiten Elementposition um eine Drehachse drehbar ist, die im Wesentlichen zu einer Achse der Kammer (102) senkrecht ist;
gegebenenfalls wobei die Drehachse mit jedem des ersten Durchgangs (P1) und des zweiten Durchgangs (P2) unausgerichtet ist, wenn sich das bewegliche Element (232) in der ersten Elementposition befindet und wenn sich das bewegliche Element (232) in der zweiten Elementposition befindet.

4. Vorrichtung zur Bereitstellung eines nicht brennbaren Aerosols (100) nach einem der Ansprüche 2 oder 3, wobei der Mechanismus (114) eine bewegliche Basis (234a) der Kammer (102) zum Anpassen der Länge der Kammer (102) umfasst.

5. Vorrichtung zur Bereitstellung eines nicht brennbaren Aerosols (100) nach Anspruch 4, wobei der Mechanismus (114) mindestens eine mechanische Verbindung (234b, 234c) umfasst, die das bewegliche Element (232) mit der beweglichen Basis (234a) der Kammer (102) verbindet.

6. Vorrichtung zur Bereitstellung eines nicht brennbaren Aerosols (100) nach Anspruch 5, wobei die mindestens eine mechanische Verbindung (234b, 234c) so angeordnet ist, dass Bewegung der beweglichen Basis (234a) von einer ersten Basisposition in eine zweite Basisposition bewirkt, dass sich das bewegliche Element (232) von der ersten Elementposition in die zweite Elementposition bewegt.

7. Vorrichtung zur Bereitstellung eines nicht brennbaren Aerosols (100) nach einem der Ansprüche 4 bis 6, wobei der Mechanismus (114) lineare Bewegung der beweglichen Basis (234a) in Drehbewegung des beweglichen Elements (232) umwandelt.

8. Vorrichtung zur Bereitstellung eines nicht brennbaren Aerosols (100) nach einem der Ansprüche 4 bis 7, die weiter eine Öffnung (268) zu einer Vertiefung (270) an einem distalen Ende der Vorrichtung zur Bereitstellung eines nicht brennbaren Aerosols (100) umfasst, wobei die Vertiefung (270) so konfiguriert ist, dass sie einen ersten entfernbaren Einsatz (254) aufnimmt, der von einem Benutzer durch die Öffnung (268) in die Vertiefung (270) eingesetzt werden kann, um die bewegliche Basis (234a) von der ersten Basisposition in die zweite Basisposition zu schieben;
gegebenenfalls weiter den ersten entfernbaren Einsatz (254) umfassend, der innerhalb der Vertiefung (270) aufgenommen ist, wobei der erste entfernbare Einsatz (254) ein Material umfasst, das Kondensat, das sich bildet, wenn die Vorrichtung zur Bereitstellung eines nicht brennbaren Aerosols (100) in Gebrauch ist, in der Vertiefung (270) sammelt.

9. Vorrichtung zur Bereitstellung eines nicht brennbaren Aerosols (100) nach einem der Ansprüche 1 bis 8, wobei die zweite Länge (L2) der Kammer (102) größer ist als die erste Länge (L1) der Kammer (102).

10. Vorrichtung zur Bereitstellung eines nicht brennbaren Aerosols (100) nach einem der Ansprüche 1 bis 9, wobei die zweite Breite (D2) kleiner ist als die erste Breite (D1).

11. Vorrichtung zur Bereitstellung eines nicht brennbaren Aerosols (100) zum Erzeugen eines Aerosols aus einem Verbrauchsmaterial (C), das Aerosol erzeugendes Material umfasst, wobei die Vorrichtung zur Bereitstellung eines nicht brennbaren Aerosols (100) umfasst:
eine Kammer (102) zum Aufnehmen eines Verbrauchsmaterials (C), das Aerosol erzeugendes Material umfasst, wobei die Kammer (102) einen Einlass (103) aufweist, der eine Breite aufweist, die angepasst werden kann; und
einen Mechanismus (114) zum Konfigurieren der Kammer (102) in mindestens einer ersten Konfiguration und einer zweiten Konfiguration;
wobei, wenn sich die Kammer (102) in der ersten Konfiguration befindet, die Breite des Einlasses (103) eine erste Breite (D1) ist, und wenn sich die Kammer (102) in der zweiten Konfiguration befindet, die Breite des Einlasses (103) eine zweite Breite (D2) ist, die sich von der ersten Breite (D1) unterscheidet, wobei der Mechanismus (114) ein bewegliches Element (232) umfasst,
**dadurch gekennzeichnet, dass**
das bewegliche Element (232) einen ersten Durchgang (P1), der die erste Breite (D1) umfasst, und einen zweiten Durchgang (P2) definiert, der die zweite Breite (D2) umfasst, und wobei der Mechanismus (114) so angeordnet ist, dass er das bewegliche Element (232) zwischen einer ersten Elementposition, die der ersten Konfiguration entspricht, und einer zweiten Elementposition, die der zweiten Konfiguration entspricht, bewegt, und wobei, wenn sich das bewegliche Element (232) in der ersten Elementposition befindet, der erste Durchgang (P1) den Einlass (103) der Kammer (102) definiert, und wenn sich das bewegliche Element (232) in der zweiten Elementposition befindet, der zweite Durchgang (P2) den Einlass (103) der Kammer (102) definiert.

12. Vorrichtung zur Bereitstellung eines nicht brennbaren Aerosols (100) nach Anspruch 11, wobei der erste Durchgang (P1) und der zweite Durchgang (P2) einander in dem beweglichen Element (232) kreuzen.

13. Vorrichtung zur Bereitstellung eines nicht brennbaren Aerosols (100) nach Anspruch 11 oder Anspruch 12, wobei das bewegliche Element (232) ein drehbares Element ist, das so angeordnet ist, dass es sich relativ zu der Kammer (102) zwischen der ersten Elementposition und der zweiten Elementposition dreht;
gegebenenfalls wobei das drehbare Element (232) zwischen der ersten Elementposition und der zweiten Elementposition um eine Drehachse drehbar ist, die im Wesentlichen senkrecht zu einer Achse der Kammer (102) ist;
gegebenenfalls wobei die Drehachse mit jedem des ersten Durchgangs (P1) und des zweiten Durchgangs (P2) unausgerichtet ist, wenn sich das bewegliche Element (232) in der ersten Elementposition befindet und wenn sich das bewegliche Element (232) in der zweiten Elementposition befindet.

14. Vorrichtung zur Bereitstellung eines nicht brennbaren Aerosols (100) nach einem der Ansprüche 11 bis 13, wobei die Kammer (102) weiter eine anpassbare Länge umfasst und der Mechanismus (114) eine bewegliche Basis (234a) der Kammer (102) zum Anpassen der Länge der Kammer (102) umfasst;
gegebenenfalls wobei der Mechanismus (114) mindestens eine mechanische Verbindung (234b, 234c) umfasst, die das bewegliche Element (232) mit der beweglichen Basis (234a) der Kammer verbindet;
gegebenenfalls wobei die mindestens eine mechanische Verbindung (234b, 234c) so angeordnet ist, dass Bewegung der beweglichen Basis (234a) von einer ersten Basisposition in eine zweite Basisposition bewirkt, dass sich das bewegliche Element (232) von der ersten Elementposition in die zweite Elementposition bewegt.

15. System zur Bereitstellung eines nicht brennbaren Aerosols, umfassend:
eine Vorrichtung zur Bereitstellung eines nicht brennbaren Aerosols (100) nach einem der Ansprüche 1 bis 14; und
mindestens ein Verbrauchsmaterial (C), das Aerosol erzeugendes Material umfasst, wobei das Verbrauchsmaterial (C) dafür bestimmt ist, in der Kammer (102) aufgenommen zu werden.

## Revendications

1. Dispositif de fourniture d'aérosol non combustible (100) pour générer un aérosol à partir d'un consommable (C) comprenant un matériau générateur d'aérosol, le dispositif de fourniture d'aérosol non combustible (100) comprenant :
une chambre (102) destinée à recevoir un consommable (C) comprenant un matériau générateur d'aérosol, la chambre (102) présentant une entrée (103) et une longueur, dans lequel l'entrée (103) présente une largeur et dans lequel la largeur de l'entrée (103) et la longueur de la chambre (102) sont ajustables ; et
un mécanisme (114) destiné à configurer la chambre (102) dans au moins une première configuration et une seconde configuration ;
dans lequel, lorsque la chambre (102) est dans la première configuration, la largeur de l'entrée (103) est une première largeur (D1) et la longueur de la chambre (102) est une première longueur (L1), et, lorsque la chambre (102) est dans la seconde configuration, la largeur de l'entrée (103) est une seconde largeur (D2) qui est différente de la première largeur (D1) et la longueur de la chambre (102) est une seconde longueur (L2) qui est différente de la première longueur (L1) ;
**caractérisé en ce que** le mécanisme (114) comprend une liaison mobile (234b, 234c) et dans lequel le mécanisme (114) est configuré de telle sorte qu'un seul mouvement de la liaison mobile (234b, 234c) amène la chambre (102) à changer entre la première configuration et la seconde configuration.

2. Dispositif de fourniture d'aérosol non combustible (100) selon la revendication 1, dans lequel le mécanisme (114) comprend un élément mobile (232), l'élément mobile (232) définissant un premier passage (P1) comprenant la première largeur (D1) et un second passage (P2) comprenant la seconde largeur (D2) et dans lequel le mécanisme (114) est conçu pour déplacer l'élément mobile (232) entre une première position d'élément correspondant à la première configuration et une seconde position d'élément correspondant à la seconde configuration et dans lequel, lorsque l'élément mobile (232) est dans la première position d'élément, le premier passage (P1) définit l'entrée (103) de la chambre (102) et, lorsque l'élément mobile (232) est dans la seconde position d'élément, le second passage (P2) définit l'entrée (103) de la chambre (102) ;
facultativement dans lequel le premier passage (P1) et le second passage (P2) se croisent dans l'élément mobile (232).

3. Dispositif de fourniture d'aérosol non combustible (100) selon la revendication 2, dans lequel l'élément mobile (232) est un élément rotatif conçu pour tourner par rapport à la chambre (102) entre la première position d'élément et la seconde position d'élément ;
facultativement dans lequel l'élément rotatif peut tourner entre la première position d'élément et la seconde position d'élément autour d'un axe de rotation qui est sensiblement perpendiculaire à un axe de la chambre (102) ;
facultativement dans lequel l'axe de rotation est désaligné avec chacun du premier passage (P1) et du second passage (P2) lorsque l'élément mobile (232) est dans la première position d'élément et lorsque l'élément mobile (232) est dans la seconde position d'élément.

4. Dispositif de fourniture d'aérosol non combustible (100) selon l'une quelconque des revendications 2 ou 3, dans lequel le mécanisme (114) comprend une base mobile (234a) de la chambre (102) pour ajuster la longueur de la chambre (102).

5. Dispositif de fourniture d'aérosol non combustible (100) selon la revendication 4, dans lequel le mécanisme (114) comprend au moins une liaison mécanique (234b, 234c) reliant l'élément mobile (232) à la base mobile (234a) de la chambre (102).

6. Dispositif de fourniture d'aérosol non combustible (100) selon la revendication 5, dans lequel la au moins une liaison mécanique (234b, 234c) est agencée de telle sorte qu'un déplacement de la base mobile (234a) d'une première position de base à une seconde position de base amène l'élément mobile (232) à se déplacer de la première position d'élément à la seconde position d'élément.

7. Dispositif de fourniture d'aérosol non combustible (100) selon l'une quelconque des revendications 4 à 6, dans lequel le mécanisme (114) convertit un mouvement linéaire de la base mobile (234a) en un mouvement de rotation de l'élément mobile (232).

8. Dispositif de fourniture d'aérosol non combustible (100) selon l'une quelconque des revendications 4 à 7, comprenant en outre une ouverture (268) donnant sur un évidement (270) au niveau d'une extrémité distale du dispositif de fourniture d'aérosol non combustible (100), dans lequel l'évidement (270) est configuré pour recevoir un première pièce d'insertion amovible (254) qui peut être insérée par un utilisateur dans l'évidement (270) par l'ouverture (268) pour pousser la base mobile (234a) de la première position de base à la seconde position de base ;
comprenant facultativement en outre la première pièce d'insertion amovible (254) reçue dans l'évidement (270), dans lequel la première pièce d'insertion amovible (254) comprend un matériau qui collecte un condensat dans l'évidement (270) qui se forme lorsque le dispositif de fourniture d'aérosol non combustible (100) est utilisé.

9. Dispositif de fourniture d'aérosol non combustible (100) selon l'une quelconque des revendications 1 à 8, dans lequel la seconde longueur (L2) de la chambre (102) est supérieure à la première longueur (L1) de la chambre (102).

10. Dispositif de fourniture d'aérosol non combustible (100) selon l'une quelconque des revendications 1 à 9, dans lequel la seconde largeur (D2) est plus petite que la première largeur (D1).

11. Dispositif de fourniture d'aérosol non combustible (100) pour générer un aérosol à partir d'un consommable (C) comprenant un matériau générateur d'aérosol, le dispositif de fourniture d'aérosol non combustible (100) comprenant :
une chambre (102) destinée à recevoir un consommable (C) comprenant un matériau générateur d'aérosol, dans lequel la chambre (102) présente une entrée (103) qui présente une largeur qui est ajustable ; et
un mécanisme (114) destiné à configurer la chambre (102) dans au moins une première configuration et une seconde configuration ;
dans lequel, lorsque la chambre (102) est dans la première configuration, la largeur de l'entrée (103) est une première largeur (D1) et lorsque la chambre (102) est dans la seconde configuration, la largeur de l'entrée (103) est une seconde largeur (D2) qui est différente de la première largeur (D1), dans lequel le mécanisme (114) comprend un élément mobile (232),
**caractérisé en ce que**
l'élément mobile (232) définit un premier passage (P1) comprenant la première largeur (D1) et un second passage (P2) comprenant la seconde largeur (D2) et dans lequel le mécanisme (114) est conçu pour déplacer l'élément mobile (232) entre une première position d'élément correspondant à la première configuration et une seconde position d'élément correspondant à la seconde configuration, et dans lequel, lorsque l'élément mobile (232) est dans la première position d'élément, le premier passage (P1) définit l'entrée (103) de la chambre (102) et, lorsque l'élément mobile (232) est dans la seconde position d'élément, le second passage (P2) définit l'entrée (103) de la chambre (102).

12. Dispositif de fourniture d'aérosol non combustible (100) selon la revendication 11, dans lequel le premier passage (P1) et le second passage (P2) se croisent dans l'élément mobile (232).

13. Dispositif de fourniture d'aérosol non combustible (100) selon la revendication 11 ou la revendication 12, dans lequel l'élément mobile (232) est un élément rotatif conçu pour tourner par rapport à la chambre (102) entre la première position d'élément et la seconde position d'élément ;
facultativement dans lequel l'élément rotatif (232) peut tourner entre la première position d'élément et la seconde position d'élément autour d'un axe de rotation qui est sensiblement perpendiculaire à un axe de la chambre (102) ;
facultativement dans lequel l'axe de rotation est désaligné avec chacun du premier passage (P1) et du second passage (P2) lorsque l'élément mobile (232) est dans la première position d'élément et lorsque l'élément mobile (232) est dans la seconde position d'élément.

14. Dispositif de fourniture d'aérosol non combustible (100) selon l'une quelconque des revendications 11 à 13, dans lequel la chambre (102) comprend en outre une longueur ajustable et le mécanisme (114) comprend une base mobile (234a) de la chambre (102) pour ajuster la longueur de la chambre (102) ;
facultativement dans lequel le mécanisme (114) comprend au moins une liaison mécanique (234b, 234c) reliant l'élément mobile (232) à la base mobile (234a) de la chambre ;
facultativement dans lequel la au moins une liaison mécanique (234b, 234c) est agencée de telle sorte qu'un déplacement mouvement de la base mobile (234a) d'une première position de base à une seconde position de base amène l'élément mobile (232) à se déplacer de la première position d'élément à la seconde position d'élément.

15. Système de fourniture d'aérosol non combustible, comprenant :
un dispositif de fourniture d'aérosol non combustible (100) selon l'une quelconque des revendications 1 à 14 ; et
au moins un consommable (C) comprenant un matériau générateur d'aérosol, le consommable (C) étant destiné à être reçu dans la chambre (102).
